# EUROPEAN PATENT APPLICATION

(11) **EP 4 570 215 A1**
(43) Date of publication of application: **18.06.2025**
(21) Application number: 23851583.7
(22) Date of filing: 24.07.2023
(51) Int. Cl.: A61F 2/07

(54) **CONVEYING APPARATUS, STENT, AND STENT SYSTEM**

(30) Priority: 11.08.2022 CN 202210958343
(71) Applicant: Shanghai MicroPort Endovascular MedTech (Group) Co., Ltd., Shanghai 201318 (CN)
(72) Inventor: MA, Mingjie, Shanghai 201318 (CN); ZHAO, Mingjie, Shanghai 201318 (CN); YAO, Yu, Shanghai 201318 (CN); ZHU, Yongfeng, Shanghai 201318 (CN)
(74) Representative: Marks & Clerk LLP
(86) International application number: PCT/CN2023/108906
(87) International publication number: WO 2024/032361

(57) **Abstract**

The present application relates to a conveying apparatus, a stent, and a stent system. An outer sheath (1120) movably sleeves an inner tube (1110). A first guide wire (1300) is movably inserted between the inner tube (1110) and the outer sheath (1120). The first guide wire (1300) is located on a small bending side of a sleeve assembly (1100). The first guide wire (1300) is connected to the inner tube (1110). A constraint member (1200) connects the first guide wire (1300) and the sleeve assembly (1100) to a small bending side of the stent (2100).

## Description

### CROSS REFERENCE TO RELATED APPLICATION

The present application claims priority to Chinese Patent Application with No. 202210958343.3, entitled "Conveying Apparatus, Stent, and Stent System", and filed on August 11, 2022, the content of which is expressly incorporated herein by reference in its entirety.

### TECHNICAL FIELD

The present invention relates to the field of medical equipment technology, particularly to a conveying device, a stent, and a stent system.

### BACKGROUND

Thoracic endovascular aortic repair (TEVAR) is an effective means for treating aortic dissection, which has significant advantages such as less trauma and fewer complications, etc., compared to the open surgery, and thus is developed rapidly in recent years. The TEVAR is mainly performed by sealing the proximal intima rupture of the aorta, thereby improving the blood supply to the true lumen, reducing the pressure of the false lumen, inducing the formation of false lumen thrombosis, resulting in the enlargement of the true lumen of the aorta, the formation of thrombus in the false lumen, the contraction of the false lumen and eventually the occlusion of the false lumen, and then completing the aortic arch vascular remodeling.

Due to the natural curved structure of the aortic arch and the uncertain position of the opening, it is difficult for a stent to adhere well to an inner wall of the artery, especially for patients with a sharp aortic arch radian. The phenomenon of a wedge-shaped gap between the stent and the blood vessel wall due to incomplete proximal attachment is referred to as the "bird's beak" effect. Studies have shown that the appearance and shape of the "bird's beak" are significantly correlated with the risks of type Ia and type IIa arterial endoleaks. In patients with the type Ia and type IIa arterial endoleaks, an average length of the "bird's beak" is significantly greater than that in patients without the arterial endoleak.

In related technologies, the mode of adjusting the "bird's beak" is usually synchronized with the release of the stent. After the stent is fully released, whether there is a "bird's beak" and how big the "bird's beak" is are determined and cannot be adjusted. Accordingly, the "bird's beak" cannot be completely eliminated and there is a risk of postoperative complications.

### SUMMARY

According to the embodiments of the present invention, a conveying device, a stent, and a stent system are provided.

The present application provides a conveying device, including:
a sleeve assembly, including an inner tube and an outer sheath, the outer sheath being movably sleeved outside the inner tube;
a first guide wire, movably penetrating through a space between the inner tube and the outer sheath in an axial direction of the sleeve assembly, the first guide wire being located on a inner curvature side of the sleeve assembly, and a proximal end of the first guide wire being configured to be connected to a proximal end of the inner tube; and
a constraint member, configured to connect the first guide wire to a proximal end of the inner curvature side of the sleeve assembly, and connect the first guide wire to a proximal end of a inner curvature side of a stent.

In an embodiment, the constraint member is provided on the sleeve assembly.

In an embodiment, the constraint member is provided with a first end and a second end, the first end of the constraint member is connected to the inner tube, the second end of the constraint member is provided with a first connecting portion, the constraint member is configured to penetrate through the proximal end of the inner curvature side of the stent, and is further configured to be connected to the first guide wire by using the first connecting portion.

In an embodiment, the first end of the constraint member is movably connected to the inner tube.

In an embodiment, the first end of the constraint member is movable in a circumferential direction of the inner tube.

In an embodiment, the constraint member is provided with a developing portion.

In an embodiment, the constraint member has a linear structure, and a length of the constraint member is L, satisfying 1.31D≤L≤1.45D, where D represents a diameter of the proximal end of the stent.

In an embodiment, the conveying device includes:
a second guide wire, movably penetrating through the space between the inner tube and the outer sheath in the axial direction of the sleeve assembly, the second guide wire is located on the outer curvature side of the sleeve assembly.

In an embodiment, the sleeve assembly includes:
a fixing member provided on the inner tube, the fixing member being provided with a through hole, and the number of through holes being greater than or equal to the number of first guide wires.

In an embodiment, there exist two fixing members provided in an axial direction of the inner tube, and the constraint member is located between the two fixing members.

In an embodiment, the proximal end of the inner tube is provided with a guide head, and the proximal end of the first guide wire is configured to be connected to the guide head.

The present application provides a stent, adapted to being assembled in the above-mentioned conveying device, the stent includes:
a stent body;
a covering, provided on the stent body; and
a second connecting portion, provided at a proximal end of a inner curvature side of the stent body and configured to be connected to the constraint member of the conveying device.

In an embodiment, there exist two second connecting portions which are circumferentially and symmetrically provided at the proximal end of the inner curvature side of the stent body along a central axis of the stent body.

In an embodiment, the conveying device includes a plurality of first guide wires and a plurality of constraint members, the number of the second connecting portions is equal to the number of the constraint members, and the number of the second connecting portions is greater than or equal to the number of the first guide wires.

The present application provides a stent system, including:
the conveying device; and
the stent, the conveying device being configured to be assembled with and convey the stent.

The details of one or more embodiments of the present invention are set forth in the accompanying drawings and the descriptions below, and other features, purposes, and advantages of the present application will be obvious from the description, the drawings, and the claims.

### BRIEF DESCRIPTION OF THE DRAWINGS

In order to more clearly illustrate the technical solution in the embodiments of the present invention or in the conventional technology, accompanying drawings required for descriptions in the embodiments or the conventional technology are briefly introduced below. Obviously, the drawings described below are merely embodiments of the present invention, and those skilled in the art can obtain other drawings based on the disclosed drawings without any creative effort.
FIG. 1 is a schematic structure diagram of an aortic arch vascular stent graft producing a "bird's beak" effect in related technologies.
FIG. 2 is a schematic structure diagram of a conveying device according to some embodiments of the present invention.
FIG. 3 is a schematic structure diagram of a stent according to some embodiments of the present invention.
FIG. 4 is a schematic diagram illustrating an assembly structure of a conveying device and a stent according to some embodiments of the present invention.
FIG. 5 is a schematic structure diagram of a stent with a "bird's beak" effect eliminated according to some embodiments of the present invention.
FIG. 6 is a schematic diagram illustrating a matching structure between a proximal diameter *D* of a stent and a length *L* of a constraint member according to some embodiments of the present invention.
FIG. 7 is a schematic structure diagram of a proximal end of a stent which is folded caused by a constraint member with an excessive length according to some embodiments of the present invention.
FIG. 8 is a schematic structure diagram of an active adjustment of a constraint member with an excessive length according to some embodiments of the present invention.
FIG. 9 is a schematic structure diagram of a proximal end of a stent with a "bird's beak" effect formed due to a constraint member with a too short length according to some embodiments of the present invention.
FIG. 10 is a schematic structure diagram of an active adjustment of a constraint member with a too short length according to some embodiments of the present invention.

### Reference sings:

0001, aorta;
1100, sleeve assembly; 1200, constraint member; 1300, first guide wire; 1400, second guide wire; 1500, fixing member;
1110, inner tube; 1120, outer sheath; 1130, guide head;
1210, first connecting portion;
2100, stent body; 2200, covering; 2300, second connecting portion.

### DETAILED DESCRIPTION

The technical solution in the embodiments of the present invention will be clearly and completely described below in conjunction with the accompanying drawings in the embodiments of the present invention. Obviously, the described embodiments are merely some embodiments of the present invention, rather than all of the embodiments. Based on the embodiments in the present invention, all other embodiments obtained by those skilled in the art without any creative effort shall fall within the scope of protection of the present application.

In the description, the terms "proximal end" and "distal end" refer to the relative orientation, position, and direction of components or actions with respect to each other from the perspective of a doctor using a medical device. Although the terms "proximal end" and "distal end" are not restrictive, the "distal end" usually refers to an end of the medical device that is adjacent to the doctor during a normal operation, while the "proximal end" usually refers to an end of the medical device that first enters the patient's body.

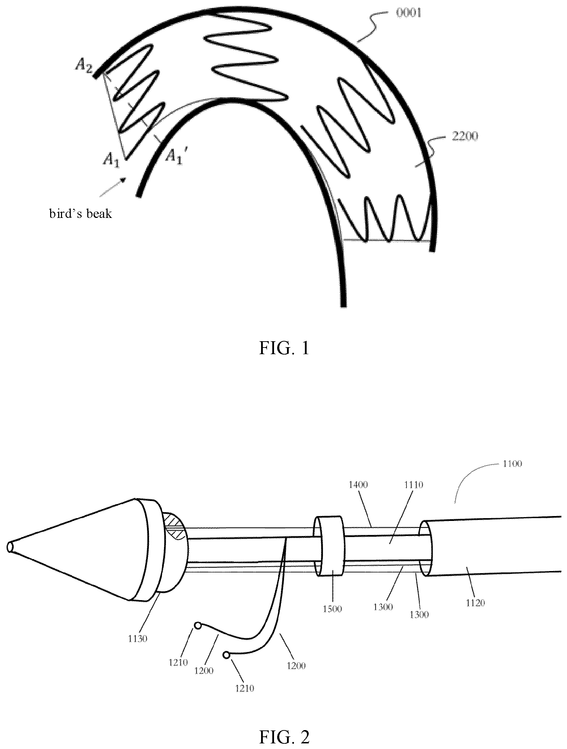

As shown in FIG. 1, an aorta 0001 in a human body has an asymmetric structure, which includes an outer curvature side and an inner curvature side. As shown in FIG. 1, an upper portion of the aorta 0001 in FIG. 1 is the outer curvature side, which has a larger curved path, and a lower portion of the aorta 0001 in FIG. 1 is the inner curvature side, which is located at an inner side of the outer curvature side and has a smaller curved path.

An aortic arch vascular stent graft usually mainly consists of a covering 2200 and a metal wire (stent body), and the metal wire is fixed to the covering 2200 by sutures. An initial state of the aortic arch vascular stent graft is a symmetrical cylindrical structure with a certain bending strength. When the aortic arch vascular stent graft is bent, there is a stress accumulation inside the aortic arch vascular stent graft, which is generally referred to as a straightening force. The straightening force may cause the proximal and distal ends of the aortic arch vascular stent graft to always tend to become into a straight state.

When the aortic arch vascular stent graft is implanted into the aortic arch portion, the aortic arch vascular stent graft may conform to the aorta 0001 and become an asymmetric structure due to the limitation of the original shape of the aorta 0001. In a portion of the aortic arch vascular stent graft sticking to the outer curvature side of the aorta 0001, the covering 2200 between the metal wires is in an unfolded state, while in a portion of the aortic arch vascular stent graft sticking to the inner curvature side of the aorta 0001, the covering 2200 between the metal wires may be compressed and folded due to the limitation of the aorta 0001.

When released, the proximal end of the aortic arch vascular stent graft is in a relatively free state due to the loss of constrained force, so that the proximal end of the aortic arch vascular stent graft (especially the inner curvature side) may bounce toward a proximal end of the blood vessel. The aortic arch vascular stent graft has a straightening force in the curved vascular lumen, which may force the proximal end of the aortic arch vascular stent graft to tilt toward the outer curvature side, that is, a point A1 cannot be attached to a point A1' on the vascular wall on the inner curvature side of the aorta 0001, such that a bird's beak is formed at the proximal end of the aortic arch vascular stent graft. This is the reason for the generation of the "bird's beak" effect. Usually, the outer curvature side A2 of the aortic arch vascular stent graft may stick to the vascular wall on the outer curvature side of the aorta 0001.

In order to eliminate the "bird's beak" effect, the present application provides the following technical solution.

Referring to FIGS. 2 to 5, in an embodiment of the present invention, a conveying device is provided. The conveying device may include a sleeve assembly 1100, a first guide wire 1300, and a constraint member 1200. The sleeve assembly 1100 may include an inner tube 1110 and an outer sheath 1120. The outer sheath 1120 is movably sleeved outside the inner tube 1110, the first guide wire 1300 movably penetrates through a space between the inner tube 1110 and the outer sheath 1120 in an axial direction of the sleeve assembly 1100. The first guide wire 1300 is located on the inner curvature side of the sleeve assembly 1100, and a proximal end of the first guide wire 1300 is configured to be connected to a proximal end of the inner tube. It should be appreciated that the proximal end of the first guide wire 1300 can be directly connected to the proximal end of the inner tube or indirectly connected to the proximal end of the inner tube through other components. For example, when the proximal end of the inner tube 1110 is provided with a guide head 1130, the proximal end of the first guide wire 1300 can be connected to the guide head 1130. The constraint member 1200 is configured to connect the first guide wire 1300 to the proximal end of the inner curvature side of the sleeve assembly 1100, and to connect the first guide wire 1300 to the proximal end of the inner curvature side of the stent.

It should be noted that the outer curvature side and inner curvature side of the sleeve assembly 1100 are determined according to the outer curvature side and the inner curvature side of the aorta 0001. Before the conveying device enters the aorta 0001, the sleeve assembly 1100 of the conveying device includes the inner tube 1110 and the outer sheath 1120 both of which have a straight structural form. At the moment, there is no relative position relationship established between the sleeve assembly 1100 and the aorta 0001, and accordingly, the sleeve assembly 1100 of the conveying device does not have a definite outer curvature side and inner curvature side. After the conveying device enters the aorta 0001, the sleeve assembly 1100 of the conveying device may undergo a corresponding deformation due to the curved shape of the aorta 0001. At the moment, the sleeve assembly 1100 of the conveying device may show the curved shape, and construct the outer curvature side and the inner curvature side.

Accordingly, generally, a side of the sleeve assembly 1100 of the conveying device configured to adapt to the outer curvature side of the aorta 0001 is referred to as the outer curvature side of the sleeve assembly 1100, and a side of the sleeve assembly 1100 of the conveying device configured to adapt to the inner curvature side of the aorta 0001 is referred to as the inner curvature side of the sleeve assembly 1100. The first guide wire 1300 is located on the inner curvature side of the sleeve assembly 1100, that is, the first guide wire 1300 is located on one side of the sleeve assembly 1100, and the one side is the side of the sleeve assembly 1100 configured to adapt to the inner curvature side of the aorta 0001 after the conveying device enters the aorta 0001. When an assembly position of the first guide wire 1300 in the sleeve assembly 1100 is determined, a side on which the first guide wire 1300 is assembled in the sleeve assembly 1100 can be defined as the inner curvature side of the sleeve assembly 1100. Accordingly, it can be understood that the position of the sleeve assembly 1100 for adapting to the aorta 0001 determines a mounting position of the first guide wire 1300, and it can also be understood that the mounting position of the first guide wire 1300 in the sleeve assembly 1100 may also reversely determine the position on the sleeve assembly 1100 at which the sleeve assembly 1100 is adapted to the aorta 0001, the two positions each other and serve as a reference to each other.

The constraint member 1200 may be a single strand of wire between a first end and a second end thereof, without a gap to avoid hooking. In addition, the constraint member 1200 is preferably made of a material with a rounded and smooth surface, and needs to have a low resistance to disengagement when released to avoid a displacement of the proximal end of the stent. The constraint member 1200 may have a structure independent of the sleeve assembly 1100, or may have a structure connected to the sleeve assembly 1100 as a whole. For example, when the constraint member 1200 has a structure independent of the sleeve assembly 1100, the constraint member 1200 may connect the first guide wire 1300 and the sleeve assembly 1100 to the proximal end of the inner curvature side of the stent by tying, winding, etc., before the conveying device enters the aorta 0001, so that the first guide wire 1300 and the sleeve assembly 1100 have the capability to pull and control the proximal end of the inner curvature side of the stent. Alternatively, in an embodiment, the constraint member 1200 is provided on the sleeve assembly 1100. The constraint member 1200 and the sleeve assembly 1100 may be formed in one piece. Regardless of whether the conveying device enters the aorta 0001, the constraint member 1200 remains connected to the sleeve assembly 1100 as one piece. Such an integrated structural form can effectively avoid unnecessary hooking problems caused by the binding and releasing processes to a certain extent, and the constraint member 1200 does not need to be separately stored when not in use, and can always be kept together with the sleeve assembly 1100.

Moreover, the constraint member 1200 can be mounted on the sleeve assembly 1100 in a variety of connection forms. For example, the constraint member 1200 can be bonded or welded to the sleeve assembly 1100. At the same time, the constraint member 1200 can also be mounted on any component of the sleeve assembly 1100 as required. For example, in an embodiment, the constraint member 1200 has a first end and a second end. The first end of the constraint member 1200 can be movably connected to the inner tube 1110. For example, the first end of the constraint member 1200 can move in a circumferential direction of the inner tube 1110. The second end of the constraint member 1200 can be provided with a first connecting portion 1210, and the second end of the constraint member 1200 is not connected to any structure when not in use, so that the first connecting portion 1210 of the second end of the constraint member 1200 is not connected to any structure.

When in use, the constraint member 1200 can be utilized to penetrate through the proximal end of the inner curvature side of the stent, and the constraint member 1200 itself can be utilized to form a connection to the proximal end of the inner curvature side of the stent. The penetrating of the constraint member 1200 through the proximal end of the inner curvature side of the stent can be a movable penetrating, and the constraint member 1200 can directly or indirectly penetrate through the covering 2200 of the stent or a corresponding matching structure. When the penetrating of the constraint member 1200 through the proximal end of the inner curvature side of the stent is completed, the first connecting portion 1210 of the constraint member 1200 can continue to be connected to the first guide wire 1300, and accordingly, the constraint member 1200 is utilized to connect the first guide wire 1300 and the sleeve assembly 1100 to the proximal end of the inner curvature side of the stent. The first connecting portion 1210 may have a structure such as a coil structure, and the coil needs to be as small as possible in size to reduce the possibility of thrombus formation, and the coil needs to be as smooth as possible.

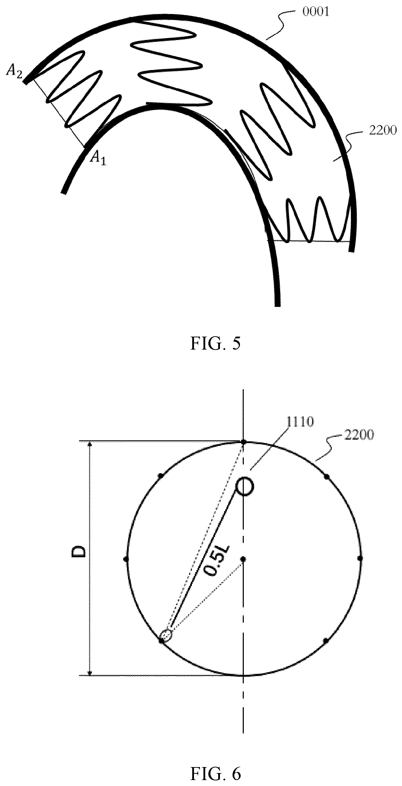

The function of the constraint member 1200 is to connect the first guide wire 1300 and the sleeve assembly 1100 to the proximal end of the inner curvature side of the stent, so that the first guide wire 1300 and the sleeve assembly 1100 can form a pull control capability on the proximal end of the inner curvature side of the stent. Accordingly, the constraint member 1200 only needs to have such a function. The constraint member 1200 may have various structural forms according to actual requirements. For example, in an embodiment, the constraint member 1200 may have a linear structure, or the constraint member 1200 may also have a belt structure, an annular structure, etc., which is not limited here. When the constraint member 1200 has the linear structure, it can be assumed that a length of the constraint member 1200 is equal to L, and a diameter of the proximal end of the stent is equal to D. Accordingly, referring to FIG. 6, the length L of the constraint member 1200 can be determined according to the diameter D of the proximal end of the stent, so that the constraint member 1200 can be adapted to select appropriate lengths when faced with stents having different sizes and structures. For example, 1.31D≤L≤1.45D, where L=1.31D, L=1.32D, L=1.33D, L =1.34D, L=1.35D, L=1.36D, L=1.37D, L=1.38D, L=1.39D, L=1.40D, L=1.41D, L=1.42D, L=1.43D, L=1.44D, L=1.45D, etc. When the length L of the constraint member 1200 is moderate, so that after the stent is released in the aorta 0001, there is no need to specially adjust the inner curvature side of the stent, so that the inner curvature side of the stent can form a good wall-adhering effect, thereby avoiding the occurrence of the "bird's beak" effect. In addition, when the stent is released in the aorta 0001, the inner tube 1110 may simultaneously be closely attached to the outer curvature side of the stent, so that the constraint member 1200 remains in a tensioned state to avoid the risk of folding of the inner curvature side of the stent.

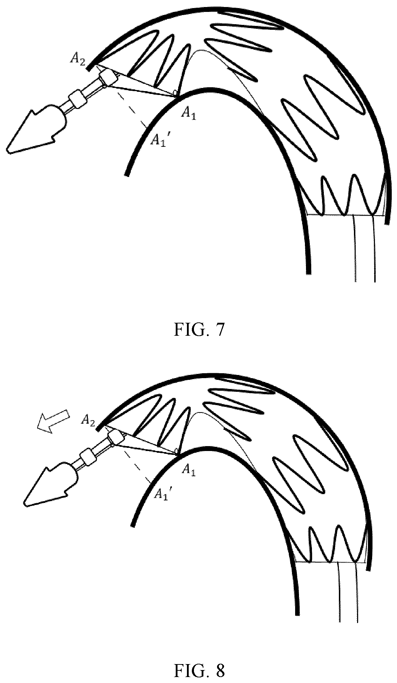

For example, referring to FIG. 7, FIG. 7 shows that if the constraint member 1200 is too long, the proximal end of the stent may be folded. If the diameter D of the proximal end of the stent can match the length L of the constraint member 1200 according to the above range, the folding can be avoided. Otherwise, referring to FIG. 8, once the folding occurs, it is necessary to move the conveying device and the stent towards the proximal so that the stent matches the shape of the aorta 0001. Such active adjustment solves the folding problem.

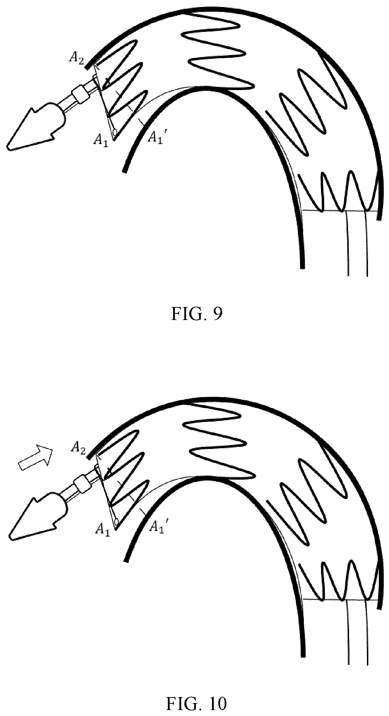

For example, referring to FIG. 9, FIG. 9 shows that if the constraint member 1200 is too short, a "bird's beak" effect may be formed at the proximal end of the stent. If the diameter D of the proximal end of the stent matches the length L of the constraint member 1200 according to the above range, the "bird's beak" effect may be avoided. Otherwise, referring to FIG. 10, once the "bird's beak" effect is encountered, it is necessary to withdraw the conveying device and the stent towards the distal, so that the stent matches the shape of the aorta 0001. Such active adjustment solves the problem of the "bird's beak" effect.

Those skilled in the art can select an appropriate matching relationship between the length L of the constraint member 1200 and the diameter D of the proximal end of the stent according to requirements, which is not limited here.

In an embodiment, the number of the first guide wires 1300 and the number of the constraint members 1200 may be one or more, for example, there exist two first guide wires 1300, which are circumferentially and symmetrically arranged on the inner curvature side of the sleeve assembly 1100 along the central axis of the conveying device.

In an embodiment, the constraint member 1200 has a developing portion which may have a structure independent of the constraint member 1200, or may be a developable structure formed in a partial structure of the constraint member 1200. For example, at least a portion of the constraint member 1200 is made of the developing material, as an example, the first connecting portion 1210 may be made of the developing material, or a portion of the constraint member 1200 adjacent to the inner tube may be made of the developing material. The developing function of the developing material may facilitate a doctor to observe a position during the adjustment process after the conveying device enters the aorta 0001.

Accordingly, since the constraint member 1200 connects the first guide wire 1300 and the sleeve assembly 1100 to the proximal end of the inner curvature side of the stent, if the stent is released in the aortic arch vessel 0001, and the proximal end of the inner curvature side of the stent has an obvious "bird's beak" phenomenon on the inner curvature side of the blood vessel wall, the conveying device can be slowly withdrawn as a whole, and the withdrawal of the conveying device can be utilized to tension the constraint member 1200. Thus, the constraint member 1200 can be utilized to drive the proximal end of the inner curvature side of the stent to move smoothly backward, so that segments of the covering 2200 on the inner curvature side of the stent are slowly stacked, thereby continuously reducing or even eliminating the "bird's beak". FIG. 5 shows the finally released stent at the moment. It should be noted that the withdrawal range of the conveying device should not be too large to avoid causing the outer curvature side of the stent to move.

When eliminating the "bird's beak" effect, the conveying device mainly adjusts the "bird's beak" effect after the stent is released in the aorta 0001. Different from most modes of synchronous adjustment when the stent is released, the above described mode is more proactive in adjustment and can flexibly adjust the "bird's beak" according to the actual size of the "bird's beak" after the stent is released, and is more targeted and can adjust the "bird's beak" to a satisfactory shape after the stent is released until the "bird's beak" is completely eliminated, thereby increasing the adaptability of the stent to the shape of the aorta 0001, expanding the range of application of the conveying device, and providing the possibility to deal with worse vascular arch shapes. In addition, the overall structure of the conveying device is simple and the production process is simple, which can achieve the optimization of the release function at a very low cost.

In addition to the first guide wire 1300, the conveying device may also include a second guide wire 1400. The second guide wire 1400 movably penetrates through the space between the inner tube 1110 and the outer sheath 1120 in the axial direction of the sleeve assembly 1100. The mounting position of the second guide wire 1400 is different from that of the first guide wire 1300, that is, the second guide wire 1400 is located on the outer curvature side of the sleeve assembly 1100, rather than the inner curvature side. Accordingly, the second guide wire 1400 can be configured to be connected to the proximal end of the outer curvature side of the stent. The second guide wire 1400 functions to prevent the stent from being washed away, fix the outer curvature side, and limit the proximal end of the outer curvature side of the stent. Accordingly, the second guide wire 1400 can tightly fix the outer curvature side of the stent on the outer curvature side of the inner tube 1110, limit the stent, and ensure that the conveying device has good positioning performance when conveying the stent.

In an embodiment, the number of the second guide wires 1400 is one or more, for example, there may exist one second guide wire 1400, as long as the outer curvature side of the stent can be fixed on the outer curvature side of the sleeve assembly.

In an embodiment, the sleeve assembly 1100 may include at least one fixing member 1500 provided on the inner tube 1110, and the fixing member 1500 is provided with a through hole, and the number of the through holes is greater than or equal to the number of the first guide wires 1300. Further, the number of the through holes is equal to a sum of the number of the first guide wires 1300 and the number of the second guide wires 1400. The through hole is configured to be passed through by the first guide wire 1300 and the second guide wire 1400, and the through hole is configured to be passed through by the first guide wire 1300 and the second guide wire 1400 simultaneously. According to the cooperation of the outer curvature side and the inner curvature side of the sleeve assembly 1100, the through hole on the fixing member 1500 can be formed at a position corresponding to the outer curvature side of the sleeve assembly 1100, or can be formed at a position corresponding to the inner curvature side of the sleeve assembly 1100, so that the formed through hole can be configured to be passed through by the first guide wire 1300 or the second guide wire 1400 respectively. The number of fixing members 1500 can be set according to requirements. For example, in an embodiment, there may exist one fixing member 1500. After penetrating through the fixing member 1500, the first guide wire 1300 or the second guide wire 1400 can be directly connected to the proximal end of the inner tube 1110 or indirectly connected to the proximal end of the inner tube 1110 through other components. In particular, when a guide head 1130 is provided at the proximal end of the inner tube 1110, the proximal end of the first guide wire 1300 or the second guide wire 1400 can be connected to the guide head 1130 to prevent the proximal end of the first guide wire 1300 or the second guide wire 1400 from being suspended.

Alternatively, there may exist two fixing members 1500 which are provided in the axial direction of the inner tube 1110, and a distance between the two adjacent fixing members 1500 can be set in a range of 2cm to 8cm. The through holes on the two fixing members 1500 are in one-to-one correspondence, in order to facilitate the passage of the first guide wire 1300 or the second guide wire 1400. The structures of the two fixing members 1500 may be the same or different, and the constraint member 1200 is located between the two fixing members 1500. The position between the two fixing members 1500 corresponds vertically to the proximal end of the inner curvature side of the stent, in order to facilitate the connection between the constraint member 1200 and the proximal end of the inner curvature side of the stent.

The guide head 1130 has a function of guiding the sleeve assembly 1100 to move in the aorta 0001 according to a predetermined trajectory. Usually, the guide head 1130 has a conical structure, that is, a proximal end of the guide head 1130 is a cone top, and a distal end of the guide head 1130 is connected to the proximal end of the inner tube 1110. The connection between the distal end of the guide head 1130 and the proximal end of the inner tube 1110 can be a same-diameter connection. When the diameter of the distal end of the guide head 1130 is the same as that of the proximal end of the inner tube 1110, the connection between the guide head 1130 and the inner tube 1110 forms a smooth connection structure. The connection between the distal end of the guide head 1130 and the proximal end of the inner tube 1110 may also be a different-diameter connection. When the diameter of the distal end of the guide head 1130 is different from that of the proximal end of the inner tube 1110, the connection between the guide head 1130 and the inner tube 1110 forms a step-shaped connection structure. At the moment, the dismeter of the distal end of the guide head 1130 may be larger than that of the proximal end of the inner tube 1110. A plurality of perforations may be formed on the guide head 1130. The perforations can be configured for guiding components such as the first guide wire 1300 or the second guide wire 1400 to penetrate therethrough, forming a connection with the guide head 1130 to implement control of corresponding actions. Those skilled in the art can configure the guide head 1130 and the perforations on the guide head 1130 according to requirements, which is not limited herein.

At the moment, when there exist two fixing members 1500, the fixing member 1500 adjacent to the proximal end of the inner tube 1110 can also be connected to the guide head 1130, so that the first guide wire 1300 or the second guide wire 1400 can be indirectly connected to the proximal end of the inner tube 1110 through the guide head after penetrating through the fixing member 1500, thereby avoiding the proximal end of the first guide wire 1300 or the second guide wire 1400 from being suspended.

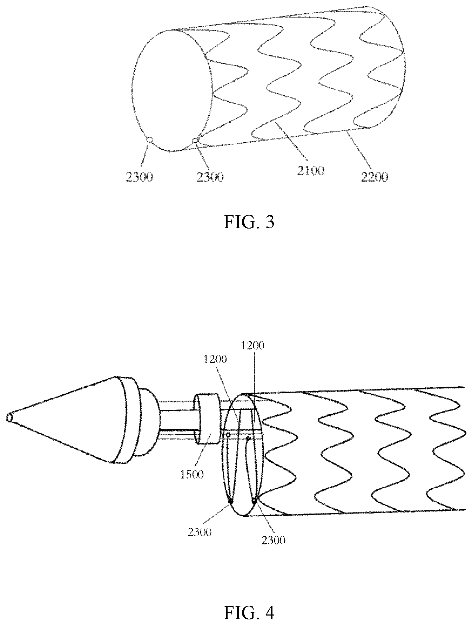

Referring to FIG. 3, the present application further provides a stent, which is adapted to be assembled to the conveying device. The stent may include a stent body 2100, a covering 2200, and a second connecting portion 2300. The covering 2200 is provided on the stent, and at least one second connecting portion 2300 is provided at the proximal end of the inner curvature side of the stent body 2100, and the second connecting portion 2300 is configured to be connected to the constraint member 1200 of the conveying device.

It should be noted that the outer curvature side and inner curvature side of the stent body 2100 (which are also equivalent to the outer curvature side and the inner curvature side of the stent) are determined according to the outer curvature side and the inner curvature side of the aorta 0001. Before entering the aorta 0001, the stent body 2100 is in a straight structural form. At the moment, the stent body 2100 does not establish a relative position relationship with the aorta 0001, and therefore the stent body 2100 does not have a definite outer curvature side and inner curvature side. After entering the aorta 0001, the stent body 2100 may be deformed accordingly due to the curve form of the aorta 0001. At the moment, the stent body 2100 may show a curve form, accordingly the outer curvature side and the inner curvature side are constructed.

Accordingly, under normal conditions, a side of the stent body 2100 configured to adapt to the outer curvature side of the aorta 0001 is referred to as the outer curvature side of the stent body 2100, and a side of the stent body 2100 configured to adapt to the inner curvature side of the aorta 0001 is referred to as the inner curvature side of the stent body 2100. The second connecting portion 2300 is provided at the proximal end of the inner curvature side of the stent body 2100, that is, the second connecting portion 2300 is located at the proximal end of one side of the stent body 2100, and the one side is the side of the stent body 2100 configured to adapt to the aorta 0001 after the stent body 2100 enters the aorta 0001. When the assembly position of the second connecting portion 2300 in the stent body 2100 is determined, a side of the stent body 2100 on which the second connecting portion 2300 is assembled in the stent body 2100 can be defined as the inner curvature side of the stent body 2100. Accordingly, it can be understood that the position on the stent body 2100 for adapting to the aorta 0001 determines the mounting position of the second connecting portion 2300, and it can also be understood that the mounting position of the second connecting portion 2300 in the stent body 2100 also reversely determines the position on the stent body 2100 in which the stent body 2100 is adapted to the aorta 0001, the two positions each other and serve as references to each other.

The second connecting portion 2300 is provided at the proximal end of the inner curvature side of the stent body 2100, so that the constraint member 1200 can have the capability to control the proximal end of the inner curvature side of the stent through the connection with the second connecting portion 2300, and when the stent is bent along the curved shape of the aorta 0001, the proximal end of the outer curvature side of the stent may closely attach to the blood vessel wall. Accordingly, the second connecting portion 2300 is located at the proximal end of the inner curvature side of the stent body 2100, rather than at the proximal end of the outer curvature side of the stent body 2100, thereby preventing the constraint member 1200 from being pressed between the blood vessel wall and the stent and unable to get out due to being connected to the proximal end of the outer curvature side of the stent after the stent is released.

The second connecting portion 2300 may have various structural forms. For example, the second connecting portion 2300 may have a coil structure, the coil needs to be as small as possible in size to reduce the possibility of thrombus formation, and the coil needs to be as smooth as possible and the length of the coil should also be small, because the coil may easily float in the blood vessel and form a thrombus if the coil is too long. The second connecting portion 2300 is located at the proximal end of the inner curvature side of the stent body 2100, and can allow the constraint member 1200 to penetrate through, so that the constraint member 1200 and the proximal end of the inner curvature side of the stent body 2100 may form a movable connection, allowing for the penetration of the constraint member 1200 through the connecting member 2300. After penetrating through the second connecting portion 2300, the constraint member 1200 can be utilized to be continuously connected to the first guide wire 1300, that is, the first guide wire 1300 penetrates through the first connecting portion 1210 of the constraint member 1200. Those skilled in the art can select the structure of the second connecting portion 2300 according to requirements, which is not limited here. The number of the second connecting portions 2300 can be selected according to requirements. Regardless of the number of the second connecting portions 2300, the second connecting portions 2300 are provided at the proximal end of the inner curvature side of the stent body 2100 and arranged in a certain form. For example, in an embodiment, there exist two second connecting portions 2300, and the two second connecting portions 2300 are circumferentially and symmetrically provided at the proximal end of the inner curvature side of the stent body 2100 along the central axis of the stent body 2100.

In an embodiment, the number of the first guide wires 1300 and the number of the constraint member 1200 of the conveying device is one or more, the number of the second connecting portions 2300 is equal to the number of the constraint members, and the number of the second connecting portions 2300 is greater than or equal to the number of the first guide wires 1300. For example, there exist two first guide wires 1300, and the two first guide wires 1300 are circumferentially and symmetrically provided on the inner curvature side of the sleeve assembly 1100 along the central axis of the conveying device. The number of the constraint members and the number of the second connecting portions are also two, and the two second connecting portions 2300 are circumferentially and symmetrically provided on the proximal end of the inner curvature side of the stent body 2100 along the central axis of the stent body 2100.

Referring to FIGS. 4 and 5, the first guide wire 1300 is located on the inner curvature side of the sleeve assembly 1100, and the second guide wire 1400 is located on the outer curvature side of the sleeve assembly 1100. The first guide wire 1300 and the second guide wire 1400 can be led out from a space between the inner tube 1110 and the outer sheath 1120, and respectively penetrate through the through holes of the fixing member 1500 adjacent to the distal end and are led out. The constraint member 1200 is fixed on the inner tube 1110, and the constraint member 1200 is located on the inner curvature side of the inner tube 1110. When the stent is mounted, the constraint member 1200 first penetrates through the proximal end of the inner curvature side of the stent, the second connecting portion 2300 can be utilized to implement the penetration connection, then the first connecting portion 1210 of the constraint member 1200 is hooked on the first guide wire 1300, and then the first guide wire 1300 penetrates through the fixing member 1500 adjacent to the proximal end and is finally fixed onto the guide head 1130. After penetrating through the fixing member 1500 adjacent to the distal end, the second guide wire 1400 can directly penetrate the proximal covering on the outer curvature side of the stent, and then pass through the fixing member 1500 adjacent to the proximal end, and then penetrate into the guide head 1130. At this point, the proximal ends of the outer curvature side and the inner curvature side of the stent can be fixed.

The adjustment of the "bird's beak" effect mainly takes effect after the stent is released. When the stent enters the aorta 0001 and bounces open, the second guide wire 1400 can be pulled out to allow the outer curvature side of the stent to fit the blood vessel wall. If the arch of the blood vessel is smooth and the operation is appropriate, the inner curvature side of the stent may not have an obvious "bird's beak" effect. In this case, the first guide wire 1300 can be directly pulled to withdraw the first guide wire 1300 and perform the complete release of the stent. However, if there is an obvious "bird's beak" phenomenon on the inner curvature side of the blood vessel, the conveying device can be slowly withdrawn as a whole, and the withdrawal of the conveying device can be utilized to tension the constraint member 1200, thus the proximal end of the inner curvature side of the stent can be driven to move smoothly backward, so that segments of the covering 2200 on the inner curvature side of the stent are slowly stacked, thereby continuously reducing or even eliminating the "bird's beak", referring to FIG. 5 for the final release form of the stent at the moment. It should be noted that the withdrawal range of the conveying device should not be too large to avoid causing the outer curvature side of the stent to move.

The present application further provides a stent system, which may include the conveying device and the stent, and the conveying device is configured to be assembled with and convey the stent. Since the structures, functional principles and technical effects of the conveying device and the stent are described in detail above, which will not be repeated here. For any technical content related to the conveying device and the stent, reference can be made to the above description.

The technical features in the above-described embodiments may be arbitrarily combined. To make the description concise, all possible combinations of the technical limitations in the above-described embodiments are not described. However, as long as there is no contradiction in combinations of these technical limitations, these combinations should be considered to be within the scope of the present invention.

The above-described embodiments merely express several implementation modes of the present invention, and the description thereof is relatively specific and detailed, but it should not be construed as limiting the scope of the present invention. It should be pointed out that, those skilled in the art can make several modifications and improvements without departing from the concept of the present invention, which all fall within the scope of protection of the present invention. Therefore, the protection scope of the present invention shall be subject to the appended claims.

## Claims

1. A conveying device, comprising:
a sleeve assembly, comprising an inner tube and an outer sheath, the outer sheath being movably sleeved outside the inner tube;
a first guide wire, movably penetrating through a space between the inner tube and the outer sheath in an axial direction of the sleeve assembly, the first guide wire being located on a inner curvature side of the sleeve assembly, and a proximal end of the first guide wire being configured to be connected to a proximal end of the inner tube; and
a constraint member, configured to connect the first guide wire to a proximal end of the inner curvature side of the sleeve assembly, and connect the first guide wire to a proximal end of a inner curvature side of a stent.

2. The conveying device according to claim 1, wherein the constraint member is provided on the sleeve assembly.

3. The conveying device according to claim 1, wherein the constraint member is provided with a first end and a second end, the first end of the constraint member is connected to the inner tube, the second end of the constraint member is provided with a first connecting portion, the constraint member is configured to penetrate through the proximal end of the inner curvature side of the stent, and is further configured to be connected to the first guide wire by using the first connecting portion.

4. The conveying device according to claim 3, wherein the first end of the constraint member is movably connected to the inner tube.

5. The conveying device according to claim 4, wherein the first end of the constraint member is movable in a circumferential direction of the inner tube.

6. The conveying device according to claim 1, wherein the constraint member is provided with a developing portion.

7. The conveying device according to claim 1, wherein the constraint member has a linear structure, and a length of the constraint member is L, satisfying 1.31D≤L≤1.45D, where D represents a diameter of the proximal end of the stent.

8. The conveying device according to claim 1, comprising:
a second guide wire, movably penetrating through the space between the inner tube and the outer sheath in the axial direction of the sleeve assembly, wherein the second guide wire is located on the outer curvature side of the sleeve assembly.

9. The conveying device according to claim 1, wherein the sleeve assembly comprises:
a fixing member provided on the inner tube, the fixing member being provided with a through hole, and the number of through holes being greater than or equal to the number of first guide wires.

10. The conveying device according to claim 9, wherein there exist two fixing members provided in an axial direction of the inner tube, and the constraint member is located between the two fixing members.

11. The conveying device according to claim 1, wherein the proximal end of the inner tube is provided with a guide head, and the proximal end of the first guide wire is configured to be connected to the guide head.

12. A stent, adapted to being assembled in the conveying device of any one of claims 1 to 11, the stent comprising:
a stent body;
a covering, provided on the stent body; and
a second connecting portion, provided at a proximal end of a inner curvature side of the stent body and configured to be connected to the constraint member of the conveying device.

13. The stent according to claim 12, wherein there exist two second connecting portions which are circumferentially and symmetrically provided at the proximal end of the inner curvature side of the stent body along a central axis of the stent body.

14. The stent according to claim 12, wherein the conveying device comprises a plurality of first guide wires and a plurality of constraint members, the number of the second connecting portions is equal to the number of the constraint members, and the number of the second connecting portions is greater than or equal to the number of the first guide wires.

15. A stent system, comprising:
the conveying device of any one of claims 1 to 11; and
the stent of any one of claims 12 to 14, wherein the conveying device is configured to be assembled with and convey the stent.
